# EUROPÄISCHE PATENTSCHRIFT

(11) **EP 4 265 190 B1**
(45) Veröffentlichungstag und Bekanntmachung des Hinweises auf die Patenterteilung: **10.09.2025**
(21) Anmeldenummer: 22199183.9
(22) Anmeldetag: 30.09.2022
(51) Int. Cl.: A61B 6/03

(54) **COMPUTERTOMOGRAPHIEGERÄT UND VERFAHREN ZUM ABDECKEN EINES ABZUDECKENDEN BEREICHS EINER GANTRY EINES COMPUTERTOMOGRAPHIEGERÄTS**
COMPUTER TOMOGRAPHY DEVICE AND METHOD FOR COVERING A COVERED AREA OF A GANTRY OF A COMPUTER TOMOGRAPHY DEVICE
APPAREIL DE TOMOGRAPHIE ASSISTÉE PAR ORDINATEUR ET PROCÉDÉ DE COUVERTURE D'UNE ZONE À RECOUVRIR D'UN PORTIQUE MOBILE D'UN APPAREIL DE TOMOGRAPHIE ASSISTÉE PAR ORDINATEUR

(43) Veröffentlichungstag der Anmeldung: 25.10.2023
(73) Patentinhaber: Siemens Healthineers AG, 91301 Forchheim (DE)
(72) Erfinder: Eichner, Christoph, 91052 Erlangen (DE); Löwen, Viktor, 53757 Sankt Augustin (DE); Müller, Hans-Jürgen, 91362 Pretzfeld (DE)
(74) Vertreter: Siemens Healthineers Patent Attorneys

(56) Entgegenhaltungen:
- WO-A1-2018/098147
- US-A- 4 645 933
- US-A1- 2018 235 552

## Beschreibung

Die Erfindung betrifft ein Computertomographiegerät. Die Erfindung betrifft ferner ein Verfahren zum Abdecken eines abzudeckenden Bereichs einer Gantry eines Computertomographiegeräts.

Bei einem stationären Computertomographiegerät bleibt die Gantry relativ zu einer Umgebung des Computertomographiegeräts während der Bildgebungsuntersuchung stationär, wobei ein Untersuchungsobjekt relativ zu der Gantry bewegt wird. In bestimmten Situation kann es vorteilhaft sein, wenn eine Scanbewegung durchgeführt werden kann, während das Untersuchungsobjekt relativ zu einer Umgebung des Computertomographiegeräts, insbesondere relativ zu einem Untersuchungsraum, ruht. Dafür wird das Projektionsdatenakquisitionssystem des Computertomographiegeräts relativ zu der Umgebung des Computertomographiegeräts translatorisch bewegt, während mittels des Projektionsdatenakquisitionssystems Projektionsdaten von einem Untersuchungsbereich des Untersuchungsobjekts erfasst werden.

Dafür kann beispielsweise vorgesehen sein, dass ein erster Gantryteil, welcher das Projektionsdatenakquisitionssystem aufweist, mittels einer Linearführung relativ zu einem zweiten Gantryteil bewegbar gelagert ist. Insbesondere im Bereich der Linearführung kann es erforderlich sein, einen Bereich der Gantry abzudecken, beispielsweise um diesen gegen einen Eingriff von außen abzusichern und/oder gegen Verunreinigungen, insbesondere gegen Staub und/oder Flüssigkeiten, abzudichten.

Als Stand der Technik sind hierbei die WO 2018/098147 A1 und US 2018/235552 A1 zu nennen.

Die Erfindung hat die Aufgabe, ein Schienensystem, das einer relativ zueinander bewegbaren Lagerung von zwei Gantryteilen eines Computertomographiegeräts dient, mittels eines flexiblen Flächengebildes möglichst dicht abzudecken. Jeder Gegenstand eines unabhängigen Anspruchs löst diese Aufgabe. In den abhängigen Ansprüchen sind weitere vorteilhafte Aspekte der Erfindung berücksichtigt.

Die Erfindung betrifft ein Computertomographiegerät, aufweisend eine Gantry mit einem ersten Gantryteil, einem zweiten Gantryteil und einem flexiblen Flächengebilde,
- wobei der erste Gantryteil einen Rotor mit einem Projektionsdatenakquisitionssystem aufweist und mittels einer Linearführung derart relativ zu dem zweiten Gantryteil bewegbar gelagert ist, dass eine Translationsbewegung des ersten Gantryteils relativ zu dem zweiten Gantryteil ausgeführt werden kann, insbesondere entlang der Linearführung ausgeführt werden kann,
- wobei die Gantry eine Spannvorrichtung für das flexible Flächengebilde aufweist und dass das flexible Flächengebilde einen abdeckenden Abschnitt aufweist, wobei der abdeckende Abschnitt zwischen dem ersten Gantryteil und dem zweiten Gantryteil zum Abdecken eines abzudeckenden Bereichs der Gantry aufgespannt ist, insbesondere mittels der Spannvorrichtung aufgespannt ist.

Die Linearführung weist ein Schienensystem und ein Wagensystem, welches mit dem Schienensystem zusammenwirkt, auf. Die Erfindung sieht vor, dass der erste Gantryteil das Wagensystem aufweist und dass der zweite Gantryteil das Schienensystem aufweist. Die Erfindung sieht vor, dass der abzudeckende Bereich das Schienensystem umfasst.

Die Gantry kann beispielsweise ferner einen Linearantrieb zum Antreiben der Translationsbewegung des ersten Gantryteils relativ zu dem zweiten Gantryteil aufweisen. Der Linearantrieb kann beispielsweise eine Gewindespindel und einen Gewindetrieb, der mit der Gewindespindel zusammenwirkt, aufweisen.

Insbesondere kann vorgesehen sein, dass der erste Gantryteil den Gewindetrieb aufweist und/oder dass der zweite Gantryteil die Gewindespindel aufweist.

Insbesondere kann vorgesehen sein, dass der abzudeckende Bereich der Gantry einen abzudeckenden Bereich des zweiten Gantryteils umfasst und/oder dass der abzudeckende Bereich der Gantry einen abzudeckenden Bereich des ersten Gantryteils umfasst. Insbesondere kann vorgesehen sein, dass der zweite Gantryteil den abzudeckenden Bereich der Gantry aufweist.

Das flexible Flächengebilde kann beispielsweise als Textil und/oder als Folie, insbesondere Kunststofffolie, ausgebildet sein. Das flexible Flächengebilde kann insbesondere in Form eines flachen Bandes ausgebildet sein. Das flexible Flächengebilde kann beispielsweise gewebt sein. Das flexible Flächengebilde kann beispielsweise imprägniert sein und/oder Verunreinigungen abweisende Oberflächen aufweisen. Das flexible Flächengebilde kann insbesondere derart ausgebildet sein, dass es mit geringem Aufwand gründlich gereinigt werden kann.

Die Erfindung sieht vor, dass die Spannvorrichtung dazu eingerichtet ist, eine Längenänderung des abdeckenden **Ab**schnitts, welche der Translationsbewegung des ersten Gantryteils relativ zu dem zweiten Gantryteil folgt, zu bewirken.

Insbesondere kann vorgesehen sein, dass der erste Gantryteil und der zweite Gantryteil relativ zueinander derart angeordnet sind, dass die Translationsbewegung des ersten Gantryteils relativ zu dem zweiten Gantryteil eine Längenänderung des abzudeckenden Bereichs der Gantry bewirkt.

Insbesondere kann vorgesehen sein, dass die Spannvorrichtung dazu eingerichtet ist, einer Veränderung einer Zugspannung in dem abdeckenden Abschnitt entgegenzuwirken, insbesondere derart entgegenzuwirken, dass die Längenänderung des abdeckenden Abschnitts der Translationsbewegung des ersten Gantryteils relativ zu dem zweiten Gantryteil ohne eine wesentliche Zugspannungsveränderung in dem abdeckenden Abschnitt folgt.

Eine Ausführungsform sieht vor, dass das flexible Flächengebilde einen zurückgezogenen Abschnitt aufweist, wobei mittels der Spannvorrichtung ein Übergang zwischen dem abdeckenden Abschnitt und dem zurückgezogenen Abschnitt ausgebildet ist, wobei die Längenänderung des abdeckenden Abschnitts erfolgt, indem ein Teilabschnitt des flexiblen Flächengebildes den Übergang zwischen dem abdeckenden Abschnitt und dem zurückgezogenen Abschnitt passiert.

Die Erfindung sieht vor, dass die Spannvorrichtung eine Wickelvorrichtung aufweist, welche für eine Wickelbewegung des flexiblen Flächengebildes eingerichtet ist, wobei die Längenänderung des abdeckenden Abschnitts mit der Wickelbewegung des flexiblen Flächengebildes einhergeht. Insbesondere kann vorgesehen sein, dass die Längenänderung des abdeckenden Abschnitts durch die Wickelbewegung des flexiblen Flächengebildes erfolgt und/oder dass der zurückgezogene Abschnitt einen mittels der Wickelvorrichtung aufgewickelten Abschnitt aufweist.

Die Wickelvorrichtung kann beispielsweise eine Welle für das flexible Flächengebilde und/oder ein Spannelement, beispielsweise in Form einer Drehfeder, aufweisen. Das Spannelement kann beispielsweise ein Spann-Drehmoment auf die Welle derart ausüben, dass über die Welle eine Zugspannung auf das flexible Flächengebilde wirkt. Die Wickelvorrichtung und das flexible Flächengebilde können beispielsweise in der Art eines Rollos zusammenwirken und/oder derart zusammenwirken, dass das flexible Flächengebilde straff gespannt, jedoch nicht überdehnt, ist.

Insbesondere kann vorgesehen sein, dass eine Verkürzung des abdeckenden Abschnitts durch ein Aufwickeln des flexiblen Flächengebildes mittels der Wickelvorrichtung erfolgt und/oder dass eine Verlängerung des abdeckenden Abschnitts durch ein Abwickeln des flexiblen Flächengebildes mittels der Wickelvorrichtung erfolgt.

Das Aufwickeln des flexiblen Flächengebildes mittels der Wickelvorrichtung kann beispielsweise mittels des Spannelements der Wickelvorrichtung angetrieben sein. Das Abwickeln des flexiblen Flächengebildes mittels der Wickelvorrichtung kann beispielsweise durch eine Zugkraft angetrieben sein, welche von dem ersten Gantryteil und dem zweiten Gantryteil auf das flexible Flächengebilde ausgeübt wird und/oder welche auf die Welle der Wickelvorrichtung ein Zug-Drehmoment ausübt, welches das Spann-Drehmoment überwindet.

Eine Ausführungsform sieht vor, dass die Spannvorrichtung dazu eingerichtet ist, einen Randbereich des abdeckenden Abschnitts hin zu dem abzudeckenden Bereich der Gantry zu drücken. Der Randbereich des abdeckenden Abschnitts kann insbesondere an dem Übergang zwischen dem abdeckenden Abschnitt und dem zurückgezogenen Abschnitt ausgebildet sein.

Eine Ausführungsform sieht vor, dass die Spannvorrichtung eine erste Umlenkrolle für das flexible Flächengebilde aufweist, wobei das flexible Flächengebilde zwischen der ersten Umlenkrolle und dem abzudeckenden Bereich der Gantry hindurchgeführt ist, wobei die erste Umlenkrolle an einer Außenseite des flexiblen Flächengebildes anliegend das flexible Flächengebilde umlenkt, wenn sich der erste Gantryteil in einem ersten Positionsbereich relativ zu dem zweiten Gantryteil befindet, wobei die Außenseite des flexiblen Flächengebildes von dem abzugrenzenden Bereich der Gantry abgewandt ist.

Insbesondere kann vorgesehen sein, dass der Übergang zwischen dem abdeckenden Abschnitt und dem zurückgezogenen Abschnitt an der ersten Umlenkrolle ausgebildet ist und/oder dass der Randbereich des abdeckenden Abschnitts an der ersten Umlenkrolle ausgebildet ist.

Eine Ausführungsform sieht vor, dass die Rollachse der ersten Umlenkrolle und die Linearführung im Wesentlichen senkrecht zueinander ausgerichtet sind.

Eine Ausführungsform sieht vor, dass die Spannvorrichtung eine Spannvorrichtungs-Tragstruktur, ein Schwenklager und einen Schwenkarm aufweist, wobei der Schwenkarm mittels des Schwenklagers relativ zu der Spannvorrichtungs-Tragstruktur um eine Schwenkachse schwenkbar gelagert ist, wobei die Rollachse der ersten Umlenkrolle zu der Schwenkachse im Wesentlichen parallel ist, wobei die erste Umlenkrolle von der Schwenkachse beabstandet an dem Schwenkarm angeordnet ist.

Insbesondere kann vorgesehen sein, dass die Spannvorrichtung ein Federelement aufweist, welches zwischen den Schwenkarm und die Spannvorrichtungs-Tragstruktur gespannt ist und auf den Schwenkarm ein Drehmoment um die Schwenkachse relativ zu der Spannvorrichtungs-Tragstruktur derart ausübt, dass die erste Umlenkrolle das flexible Flächengebilde hin zu dem abzudeckenden Bereich der Gantry drückt, wenn sich der erste Gantryteil in einem ersten Positionsbereich relativ zu dem zweiten Gantryteil befindet.

Insbesondere kann vorgesehen sein, dass das Federelement auf den Schwenkarm das Drehmoment um die Schwenkachse relativ zu der Spannvorrichtungs-Tragstruktur derart ausübt, dass die erste Umlenkrolle den Randbereich des abdeckenden Abschnitts hin zu dem abzudeckenden Bereich der Gantry drückt, wenn sich der erste Gantryteil in einem ersten Positionsbereich relativ zu dem zweiten Gantryteil befindet.

Insbesondere kann vorgesehen sein, dass die erste Umlenkrolle mittels des Schwenkarms und des Schwenklagers relativ zu der Spannvorrichtungs-Tragstruktur um die Schwenkachse schwenkbar gelagert ist.

Eine Ausführungsform sieht vor, dass die Spannvorrichtung eine zweite Umlenkrolle für das flexible Flächengebilde aufweist, wobei das flexible Flächengebilde zwischen der ersten Umlenkrolle und der zweiten Umlenkrolle hindurchgeführt ist, wobei die zweite Umlenkrolle an einer Innenseite des flexiblen Flächengebildes anliegend das flexible Flächengebilde umlenkt, wenn sich der erste Gantryteil in einem zweiten Positionsbereich relativ zu dem zweiten Gantryteil befindet, wobei die Innenseite des flexiblen Flächengebildes dem abzugrenzenden Bereich der Gantry zugewandt ist.

Insbesondere kann vorgesehen sein, dass das flexible Flächengebilde zwischen der ersten Umlenkrolle und der zweiten Umlenkrolle derart hindurchgeführt ist, dass ein Umschlagen des flexiblen Flächengebildes von der ersten Umlenkrolle zu der zweiten Umlenkrolle erfolgt, wenn der erste Gantryteil eine Grenze zwischen dem ersten Positionsbereich relativ zu dem zweiten Gantryteil und dem zweiten Positionsbereich relativ zu dem zweiten Gantryteil passiert.

Insbesondere kann vorgesehen sein, dass die Rollachse der zweiten Umlenkrolle und die Linearführung im Wesentlichen senkrecht zueinander ausgerichtet sind und/oder dass die Rollachse der zweiten Umlenkrolle zu der Rollachse der ersten Umlenkrolle im Wesentlichen parallel ist. Insbesondere kann vorgesehen sein, dass die zweite Umlenkrolle von der Schwenkachse beabstandet an dem Schwenkarm angeordnet ist und/oder dass die zweite Umlenkrolle zwischen der ersten Umlenkrolle und der Schwenkachse angeordnet ist.

Insbesondere kann ein formschlüssiger Anschlag an der Spannungsvorrichtungs-Tragstruktur vorgesehen sein, um den Schwenkarm gegen ein zu weites Auslenken infolge des Drehmoments abzusichern, beispielsweise wenn sich der erste Gantryteil in dem zweiten Positionsbereich relativ zu dem zweiten Gantryteil befindet, wobei der Gegendruck vonseiten des abzugrenzenden Bereichs wegfällt.

Insbesondere kann vorgesehen sein, dass sich der erste Gantryteil während eines ersten Abschnitts der Translationsbewegung des ersten Gantryteils relativ zu dem zweiten Gantryteil in dem ersten Positionsbereich relativ zu dem zweiten Gantryteil befindet und/oder dass sich der erste Gantryteil während eines zweiten Abschnitts der Translationsbewegung des ersten Gantryteils relativ zu dem zweiten Gantryteil in dem zweiten Positionsbereich relativ zu dem zweiten Gantryteil befindet. Insbesondere kann vorgesehen sein, dass bei der Translationsbewegung des ersten Gantryteils relativ zu dem zweiten Gantryteil der erste Gantryteil die Grenze zwischen dem ersten Positionsbereich relativ zu dem zweiten Gantryteil und dem zweiten Positionsbereich relativ zu dem zweiten Gantryteil passiert.

Eine Ausführungsform sieht vor, dass ein erstes Ende des flexiblen Flächengebildes an dem ersten Gantryteil derart befestigt ist, dass das erste Ende des flexiblen Flächengebildes der Translationsbewegung des ersten Gantryteils relativ zu dem zweiten Gantryteil folgt, und/oder dass ein zweites Ende des flexiblen Flächengebildes an dem zweiten Gantryteil derart befestigt ist, dass während der Translationsbewegung des ersten Gantryteils relativ zu dem zweiten Gantryteil das zweite Ende des flexiblen Flächengebildes relativ zu dem zweiten Gantryteil ruht.

Erfindungsgemäß weist der erste Gantryteil die Spannvorrichtung auf. Somit weist der erste Gantryteil auch die Wickelvorrichtung auf. Insbesondere kann vorgesehen sein, dass der erste Gantryteil die Spannvorrichtungs-Tragstruktur, das Schwenklager und den Schwenkarm aufweist. Das erste Ende des flexiblen Flächengebildes kann beispielsweise an der Spannvorrichtung befestigt sein, insbesondere an der Wickelvorrichtung befestigt sein, insbesondere an der Welle der Wickelvorrichtung befestigt sein.

Das zweite Ende des flexiblen Flächengebildes kann beispielsweise an einer Verkleidung des zweiten Gantryteils befestigt sein, insbesondere geklebt und/oder zwischen zwei Verkleidungsteilen eingeklemmt, sein.

Eine Ausführungsform sieht vor, dass die Gantry eine Öffnung aufweist, wobei die Öffnung derart ausgebildet ist, dass ein Untersuchungsobjekt entlang einer Systemachse der Gantry in die Öffnung eingeführt werden kann, und/oder dass die Translationsbewegung des ersten Gantryteils relativ zu dem zweiten Gantryteil entlang der Systemachse erfolgt. Insbesondere kann vorgesehen sein, dass die Linearführung parallel zu der Systemachse ausgerichtet ist. Insbesondere kann vorgesehen sein, dass der erste Gantryteil im Wesentlichen ringförmig um die Systemachse herum angeordnet ist.

Insbesondere kann vorgesehen sein, dass der erste Gantryteil eine Drehlagerung und eine Gantry-Tragstruktur aufweist und dass der Rotor mittels der Drehlagerung mit der Gantry-Tragstruktur verbunden und relativ zu der Gantry-Tragstruktur um die Systemachse drehbar gelagert ist.

Das Untersuchungsobjekt kann beispielsweise ein Körperteil, insbesondere ein menschliches oder tierisches Körperteil, oder ein Phantom sein. Das Untersuchungsobjekt kann insbesondere ein Kopf eines Menschen sein. Das Computertomographiegerät kann insbesondere als Kopf-Computertomographiegerät und/oder als mobiles Computertomographiegerät ausgebildet sein.

Die Erfindung betrifft ferner ein Verfahren zum Abdecken eines abzudeckenden Bereichs der Gantry des erfindungsgemäßen Computertomographiegeräts, wobei die Gantry einen ersten Gantryteil, einen zweiten Gantryteil, ein flexibles Flächengebilde und eine Spannvorrichtung für das flexible Flächengebilde aufweist, das Verfahren umfassend:
- ein Aufspannen eines abdeckenden Abschnitts des flexiblen Flächengebildes zwischen dem ersten Gantryteil und dem zweiten Gantryteil zum Abdecken eines abzudeckenden Bereichs der Gantry und
- ein Ausführen einer Translationsbewegung des ersten Gantryteils relativ zu dem zweiten Gantryteil, wobei der erste Gantryteil einen Rotor mit einem Projektionsdatenakquisitionssystem aufweist und mittels einer Linearführung relativ zu dem zweiten Gantryteil bewegbar gelagert ist.

Eine Ausführungsform sieht vor, dass mittels der Spannvorrichtung eine Längenänderung des abdeckenden Abschnitts bewirkt wird, wobei die Längenänderung des abdeckenden Abschnitts der Translationsbewegung des ersten Gantryteils relativ zu dem zweiten Gantryteil folgt. Insbesondere kann vorgesehen sein, dass die die Spannvorrichtung einer Veränderung einer Zugspannung in dem abdeckenden Abschnitt entgegenwirkt.

Eine Ausführungsform sieht vor, dass mittels der Spannvorrichtung ein Übergang zwischen dem abdeckenden Abschnitt und einem zurückgezogenen Abschnitt des flexiblen Flächengebildes ausgebildet wird, und/oder dass die Längenänderung des abdeckenden Abschnitts erfolgt, indem ein Teilabschnitt des flexiblen Flächengebildes den Übergang zwischen dem abdeckenden Abschnitt und dem zurückgezogenen Abschnitt passiert.

Eine Ausführungsform sieht vor, dass die Längenänderung des abdeckenden Abschnitts durch eine Wickelbewegung des flexiblen Flächengebildes mittels einer Wickelvorrichtung der Spannvorrichtung erfolgt. Eine Ausführungsform sieht vor, dass ein Randbereich des abdeckenden Abschnitts mittels der Spannvorrichtung hin zu dem abzudeckenden Bereich der Gantry gedrückt wird.

Eine Ausführungsform sieht vor, dass der erste Gantryteil eine Rückseite einer Verkleidung der Gantry aufweist, wobei die Rückseite der Verkleidung der Gantry eine Rückseite der Öffnung ringförmig umgibt, und/oder dass der dritte Gantryteil eine Vorderseite einer Verkleidung der Gantry aufweist, wobei die Vorderseite der Verkleidung der Gantry eine Vorderseite der Öffnung ringförmig umgibt.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Die Verwendung der unbestimmten Artikel "ein" bzw. "eine" schließt nicht aus, dass das betroffene Merkmal auch mehrfach vorhanden sein kann.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.
Die Fig. 1 zeigt ein Computertomographiegerät in Form eines mobilen Kopf-Computertomographiegeräts mit einer Kopfschale und einem Schulterbrett.
Die Fig. 2 zeigt eine erste Ansicht des Computertomographiegeräts.
Die Fig. 3 zeigt eine zweite Ansicht des Computertomographiegeräts.
Die Fig. 4 zeigt eine dritte Ansicht des Computertomographiegeräts.
Die Fig. 5 zeigt ein Ablaufdiagramm eines Verfahrens zum Abdecken eines abzudeckenden Bereichs einer Gantry eines Computertomographiegeräts.

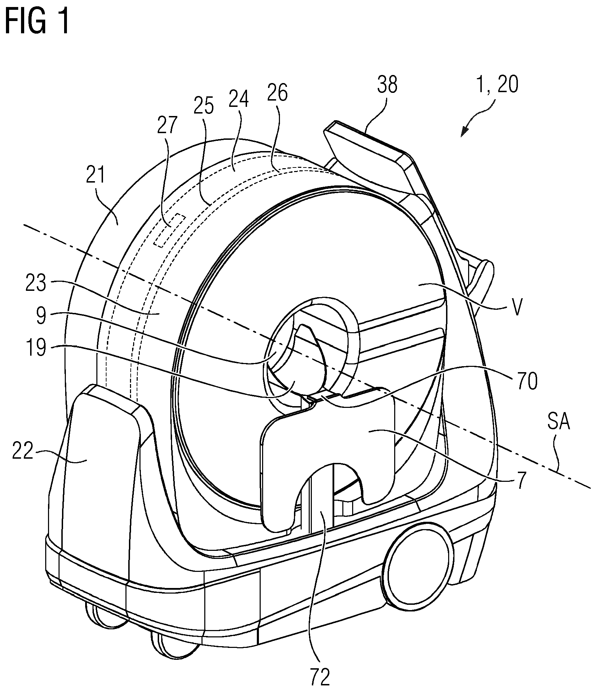

Die Fig. 1 zeigt ein Computertomographiegerät 1 in Form eines mobilen Kopf-Computertomographiegeräts mit einer Kopfschale 19 und einem Schulterbrett 7. Das Computertomographiegerät 1 weist die Gantry 20 mit einem ersten Gantryteil 21, einem zweiten Gantryteil 22 und einem dritten Gantryteil 23, wobei der erste Gantryteil 21 einen Rotor 24 mit einem Projektionsdatenakquisitionssystem 27 aufweist und mittels einer Linearführung derart relativ zu dem zweiten Gantryteil 22 bewegbar gelagert ist, dass eine Translationsbewegung des ersten Gantryteils 21 relativ zu dem zweiten Gantryteil 22 ausgeführt werden kann, insbesondere entlang der Linearführung ausgeführt werden kann.

Der erste Gantryteil 21 weist eine Drehlagerung 25 und eine Gantry-Tragstruktur 26 auf, wobei der Rotor 24 mittels der Drehlagerung 25 mit der Gantry-Tragstruktur 26 verbunden und relativ zu der Gantry-Tragstruktur 26 um die Systemachse SA drehbar gelagert ist. Der zweite Gantryteil 22 weist die Haltevorrichtung 72 auf. Die Haltevorrichtung 72 erstreckt sich entlang der vertikalen Richtung **Y.** Das Schulterbrett 7 ist mittels der Schwenkvorrichtung 70 mit der Haltevorrichtung 72 verbunden und relativ zu der Gantry 20 um eine zu der Systemachse SA senkrechte Schwenkachse schwenkbar gelagert. Die Systemachse SA ist horizontal und parallel zu der Richtung **Z.** Der zweite Gantryteil 22 weist ein Fahrwerk für eine horizontale Transportbewegung der Gantry 20 auf. Die Gantry 20 weist ferner den berührungsempfindlichen Bildschirm 38 zur Bedienung des Computertomographiegeräts 1 auf.

Die Gantry 20 weist eine Öffnung 9 auf, wobei die Öffnung 9 derart ausgebildet ist, dass ein Untersuchungsobjekt entlang einer Systemachse SA der Gantry 20 in die Öffnung 9 eingeführt werden kann, wobei die Translationsbewegung des ersten Gantryteils 21 relativ zu dem zweiten Gantryteil 22 entlang der Systemachse SA erfolgt. Insbesondere ist vorgesehen, dass der erste Gantryteil 21 im Wesentlichen ringförmig um die Systemachse SA herum angeordnet ist und dass der dritte Gantryteil 23 im Wesentlichen ringförmig um die Systemachse SA herum angeordnet ist.

Der erste Gantryteil 21 weist eine Rückseite einer Verkleidung V der Gantry 20 auf, wobei die Rückseite der Verkleidung V der Gantry 20 eine Rückseite der Öffnung 9 ringförmig umgibt. Der dritte Gantryteil 23 weist eine Vorderseite einer Verkleidung V der Gantry 20 auf, wobei die Vorderseite der Verkleidung V der Gantry 20 eine Vorderseite der Öffnung 9 ringförmig umgibt.

Neben dem gezeigten flexiblen Flächengebilde 8, welches an der Rückseite der Gantry 20 angeordnet ist, kann noch wenigstens ein weiteres flexibles Flächengebilde an der Vorderseite der Gantry 20 angeordnet sein. Beispielsweise kann für jede der zwei Schienen des Schienensystem 52 jeweils ein schmaleres flexibles Flächengebilde zu deren Abdeckung und jeweils eine kleinere Spannvorrichtung zum Aufspannen des entsprechenden schmaleren flexiblen Flächengebildes vorgesehen sein, welche insbesondere gemäß Aspekten, die für das flexible Flächengebilde und die Spannvorrichtung allgemein beschrieben wurden, ausgebildet sein können, insbesondere mit einem von der gezeigten Spannvorrichtung 84 abweichenden Funktionsumfang ausgebildet sein können.

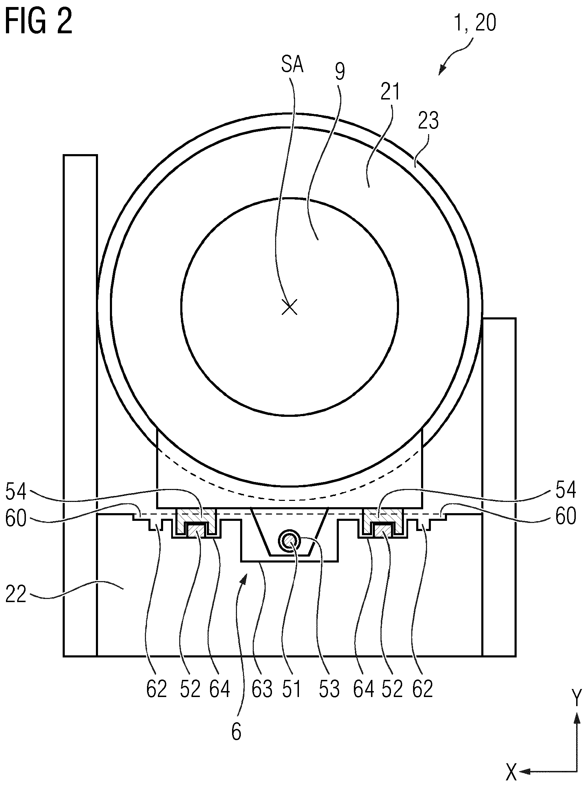

Die Fig. 2 zeigt eine erste Ansicht des Computertomographiegeräts 1. Die Linearführung weist ein Schienensystem 52 und ein Wagensystem 54, welches mit dem Schienensystem 52 zusammenwirkt, auf. Insbesondere ist vorgesehen, dass der erste Gantryteil 21 das Wagensystem 54 aufweist und dass der zweite Gantryteil 22 das Schienensystem 52 aufweist. Insbesondere ist vorgesehen, dass der abzudeckende Bereich 6 die Linearführung und damit das Schienensystem 52 umfasst.

Die Gantry 20 weist ferner einen Linearantrieb zum Antreiben der Translationsbewegung des ersten Gantryteils 21 relativ zu dem zweiten Gantryteil 22 auf. Der Linearantrieb weist beispielsweise eine Gewindespindel 51 und einen Gewindetrieb 53, der mit der Gewindespindel 51 zusammenwirkt, auf. Insbesondere ist vorgesehen, dass der erste Gantryteil 21 den Gewindetrieb 53 aufweist und dass der zweite Gantryteil 22 die Gewindespindel 51 aufweist. Insbesondere ist vorgesehen, dass der abzudeckende Bereich 6 der Gantry 20 einen abzudeckenden Bereich des zweiten Gantryteils 22 umfasst. Der abzudeckende Bereich 6 umfasst die Vertiefungen 64 für die Linearführung und eine Vertiefung 63 für den Linearantrieb.

In dem abzudeckenden Bereich 6 sind Ablaufrinnen 61, 62 angeordnet, um Verunreinigungen, insbesondere in Form von Medien, die trotz des Abdeckens in den abzudeckenden Bereich 6 gelangt sind, abzuleiten. Über den Schlauch 66 können diese Verunreinigungen aus dem zweiten Gantryteil 22 herausgeführt werden.

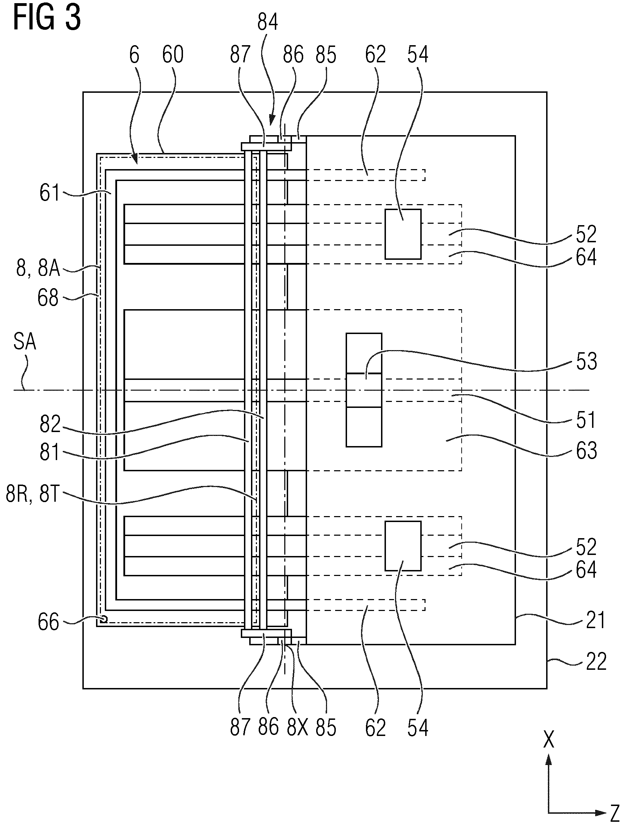

Die Fig. 3 zeigt eine zweite Ansicht des Computertomographiegeräts **1.** Das Computertomographiegerät 1 weist die Gantry 20 mit einem ersten Gantryteil 21, einem zweiten Gantryteil 22 und einem flexiblen Flächengebilde 8 auf, wobei der erste Gantryteil 21 einen Rotor 24 mit einem Projektionsdatenakquisitionssystem 27 aufweist und mittels einer Linearführung derart relativ zu dem zweiten Gantryteil 22 bewegbar gelagert ist, dass eine Translationsbewegung des ersten Gantryteils 21 relativ zu dem zweiten Gantryteil 22 ausgeführt werden kann, insbesondere entlang der Linearführung ausgeführt werden kann. Die Gantry 20 weist eine Spannvorrichtung 84 für das flexible Flächengebilde 8 auf. Das flexible Flächengebilde 8 weist einen abdeckenden Abschnitt 8A auf, wobei der abdeckende Abschnitt 8A zwischen dem ersten Gantryteil 21 und dem zweiten Gantryteil 22 zum Abdecken eines abzudeckenden Bereichs 6 der Gantry 20 aufgespannt ist, insbesondere mittels der Spannvorrichtung 84 aufgespannt ist.

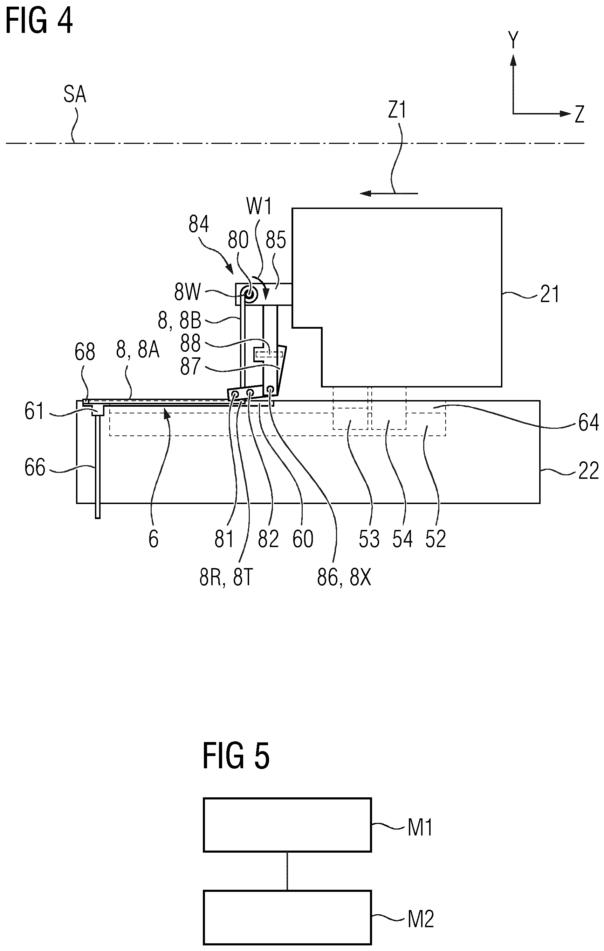

Die Fig. 4 zeigt eine dritte Ansicht des Computertomographiegeräts **1.** Die Spannvorrichtung 84 ist dazu eingerichtet, eine Längenänderung des abdeckenden Abschnitts 8A, welche der Translationsbewegung des ersten Gantryteils 21 relativ zu dem zweiten Gantryteil 22 folgt, zu bewirken. Der erste Gantryteil 21 und der zweite Gantryteil 22 sind relativ zueinander derart angeordnet, dass die Translationsbewegung des ersten Gantryteils 21 relativ zu dem zweiten Gantryteil 22 eine Längenänderung des abzudeckenden Bereichs 6 der Gantry 20 bewirkt.

Die Spannvorrichtung 84 ist dazu eingerichtet, einer Veränderung einer Zugspannung in dem abdeckenden Abschnitt 8A entgegenzuwirken, insbesondere derart entgegenzuwirken, dass die Längenänderung des abdeckenden Abschnitts 8A der Translationsbewegung des ersten Gantryteils 21 relativ zu dem zweiten Gantryteil 22 ohne eine wesentliche Zugspannungsveränderung in dem abdeckenden Abschnitt 8A folgt.

Das flexible Flächengebilde 8 weist einen zurückgezogenen **Ab**schnitt 8B auf, wobei mittels der Spannvorrichtung 84 ein Übergang 8T zwischen dem abdeckenden Abschnitt 8A und dem zurückgezogenen Abschnitt 8B ausgebildet ist, wobei die Längenänderung des abdeckenden Abschnitts 8A erfolgt, indem ein Teilabschnitt des flexiblen Flächengebildes 8 den Übergang 8T zwischen dem abdeckenden Abschnitt 8A und dem zurückgezogenen Abschnitt 8B passiert.

Die Spannvorrichtung 84 weist eine Wickelvorrichtung 80 auf, welche für eine Wickelbewegung des flexiblen Flächengebildes 8 eingerichtet ist, wobei die Längenänderung des abdeckenden Abschnitts 8A mit der Wickelbewegung des flexiblen Flächengebildes 8 einhergeht. Die Längenänderung des abdeckenden Abschnitts 8A erfolgt durch die Wickelbewegung des flexiblen Flächengebildes 8. Der zurückgezogene Abschnitt 8B weist einen mittels der Wickelvorrichtung 80 aufgewickelten Abschnitt 8W auf.

Die Spannvorrichtung 84 ist dazu eingerichtet, einen Randbereich 8R des abdeckenden Abschnitts 8A hin zu dem abzudeckenden Bereich 6 der Gantry 20 zu drücken. Der Randbereich 8R des abdeckenden Abschnitts 8A ist an dem Übergang 8T zwischen dem abdeckenden Abschnitt 8A und dem zurückgezogenen Abschnitt 8B ausgebildet.

Die Spannvorrichtung 84 weist eine erste Umlenkrolle 81 für das flexible Flächengebilde 8 auf, wobei das flexible Flächengebilde 8 zwischen der ersten Umlenkrolle 81 und dem abzudeckenden Bereich 6 der Gantry 20 hindurchgeführt ist, wobei die erste Umlenkrolle 81 an einer Außenseite des flexiblen Flächengebildes 8 anliegend das flexible Flächengebilde 8 umlenkt, wenn sich der erste Gantryteil 21 in einem ersten Positionsbereich relativ zu dem zweiten Gantryteil 22 befindet, wobei die Außenseite des flexiblen Flächengebildes 8 von dem abzudeckenden Bereich 6 der Gantry 20 abgewandt ist.

Der Übergang 8T zwischen dem abdeckenden Abschnitt 8A und dem zurückgezogenen Abschnitt 8B ist an der ersten Umlenkrolle 81 ausgebildet ist und der Randbereich 8R des abdeckenden Abschnitts 8A ist an der ersten Umlenkrolle 81 ausgebildet. Die Rollachse der ersten Umlenkrolle 81 und die Linearführung sind im Wesentlichen senkrecht zueinander ausgerichtet.

Die Spannvorrichtung 84 weist eine Spannvorrichtungs-Tragstruktur 85, ein Schwenklager 86 und einen Schwenkarm 87 auf, wobei der Schwenkarm 87 mittels des Schwenklagers 86 relativ zu der Spannvorrichtungs-Tragstruktur 85 um eine Schwenkachse 8X schwenkbar gelagert ist, wobei die Rollachse der ersten Umlenkrolle 81 zu der Schwenkachse 8X im Wesentlichen parallel ist, wobei die erste Umlenkrolle 81 von der Schwenkachse 8X beabstandet an dem Schwenkarm 87 angeordnet ist. Die Spannvorrichtung 84 weist ein Federelement 88 auf, welches zwischen den Schwenkarm 87 und die Spannvorrichtungs-Tragstruktur 85 gespannt ist und auf den Schwenkarm 87 ein Drehmoment um die Schwenkachse 8X relativ zu der Spannvorrichtungs-Tragstruktur 85 derart ausübt, dass die erste Umlenkrolle 81 das flexible Flächengebilde 8 hin zu dem abzudeckenden Bereich 6 der Gantry 20 drückt, wenn sich der erste Gantryteil 21 in einem ersten Positionsbereich relativ zu dem zweiten Gantryteil 22 befindet.

Das Federelement 88 übt auf den Schwenkarm 87 das Drehmoment um die Schwenkachse 8X relativ zu der Spannvorrichtungs-Tragstruktur 85 derart aus, dass die erste Umlenkrolle 81 den Randbereich 8R des abdeckenden Abschnitts 8A hin zu dem abzudeckenden Bereich 6 der Gantry 20 drückt, wenn sich der erste Gantryteil 21 in einem ersten Positionsbereich relativ zu dem zweiten Gantryteil 22 befindet. Insbesondere ist vorgesehen, dass die erste Umlenkrolle 81 mittels des Schwenkarms 87 und des Schwenklagers 86 relativ zu der Spannvorrichtungs-Tragstruktur 85 um die Schwenkachse 8X schwenkbar gelagert ist.

Die Spannvorrichtung 84 weist eine zweite Umlenkrolle 82 für das flexible Flächengebilde 8 auf, wobei das flexible Flächengebilde 8 zwischen der ersten Umlenkrolle 81 und der zweiten Umlenkrolle 82 hindurchgeführt ist, wobei die zweite Umlenkrolle 82 an einer Innenseite des flexiblen Flächengebildes 8 anliegend das flexible Flächengebilde 8 umlenkt, wenn sich der erste Gantryteil 21 in einem zweiten Positionsbereich relativ zu dem zweiten Gantryteil 22 befindet, wobei die Innenseite des flexiblen Flächengebildes 8 dem abzudeckenden Bereich 6 der Gantry 20 zugewandt ist.

Insbesondere ist vorgesehen, dass das flexible Flächengebilde 8 zwischen der ersten Umlenkrolle 81 und der zweiten Umlenkrolle 82 derart hindurchgeführt ist, dass ein Umschlagen des flexiblen Flächengebildes 8 von der ersten Umlenkrolle 81 zu der zweiten Umlenkrolle 82 erfolgt, wenn der erste Gantryteil 21 eine Grenze zwischen dem ersten Positionsbereich relativ zu dem zweiten Gantryteil 22 und dem zweiten Positionsbereich relativ zu dem zweiten Gantryteil 22 passiert.

Der zweite Positionsbereich relativ zu dem zweiten Gantryteil 22 folgt in der Richtung Z1 auf den ersten Positionsbereich relativ zu dem zweiten Gantryteil 21. An der Grenze zwischen dem ersten Positionsbereich relativ zu dem zweiten Gantryteil 22 und dem zweiten Positionsbereich relativ zu dem zweiten Gantryteil 22 befindet sich die erste Umlenkrolle 81 ungefähr über dem Verbindungsbereich 68.

Insbesondere ist vorgesehen, dass die Rollachse der zweiten Umlenkrolle 82 und die Linearführung im Wesentlichen senkrecht zueinander ausgerichtet sind und dass die Rollachse der zweiten Umlenkrolle 82 zu der Rollachse der ersten Umlenkrolle 81 im Wesentlichen parallel ist. Insbesondere ist vorgesehen, dass die zweite Umlenkrolle 82 von der Schwenkachse 8X beabstandet an dem Schwenkarm 87 angeordnet ist und dass die zweite Umlenkrolle 82 zwischen der ersten Umlenkrolle 81 und der Schwenkachse 8X angeordnet ist.

Ein erstes Ende des flexiblen Flächengebildes 8 ist an dem ersten Gantryteil 21 derart befestigt, dass das erste Ende des flexiblen Flächengebildes 8 der Translationsbewegung des ersten Gantryteils 21 relativ zu dem zweiten Gantryteil 22 folgt. Ein zweites Ende des flexiblen Flächengebildes 8 ist an dem zweiten Gantryteil 22 derart befestigt, dass während der Translationsbewegung des ersten Gantryteils 21 relativ zu dem zweiten Gantryteil 22 das zweite Ende des flexiblen Flächengebildes 8 relativ zu dem zweiten Gantryteil 22 ruht.

Der erste Gantryteil 21 weist die Spannvorrichtung 84 auf. Insbesondere ist vorgesehen, dass der erste Gantryteil 21 die Wickelvorrichtung 80 aufweist und dass der erste Gantryteil 21 die Spannvorrichtungs-Tragstruktur 85, das Schwenklager 86 und den Schwenkarm 87 aufweist. Das erste Ende des flexiblen Flächengebildes 8 ist an der Spannvorrichtung 84 befestigt, insbesondere an der Wickelvorrichtung 80 befestigt, insbesondere an der Welle der Wickelvorrichtung befestigt.

Somit wird das Schienensystem 52 mittels des abdeckenden Abschnitts 8A auf platzsparende Weise abgedeckt, beispielsweise um es gegen Staub und Medien abzudichten sowie vor einem Eingriff zu schützen. Wenn die Translationsbewegung des ersten Gantryteils 21 relativ zu dem zweiten Gantryteil 22 in der Richtung Z1 erfolgt, geht damit das Aufwickeln W1 des flexiblen Flächengebildes 8 mittels der Wickelvorrichtung 80 einher. Dadurch wird der abdeckende Abschnitt 8A nach und nach aus der Trajektorie der Translationsbewegung entfernt, so dass das Laufen des Wagensystems 54 auf dem Schienensystem 52 nicht durch den abdeckenden Abschnitt 8A gestört wird. Wenn die Translationsbewegung des ersten Gantryteils 21 relativ zu dem zweiten Gantryteil 22 in der Richtung Z erfolgt, geht damit ein Abwickeln des flexiblen Flächengebildes 8 von der Wickelvorrichtung 80 einher.

Mittels der Spannvorrichtung 84 wird der abdeckenden Abschnitt 8A auf den zweiten Gantryteil 22 gedrückt, insbesondere auf eine Verkleidung des zweiten Gantryteils 22 gedrückt. Der zweite Gantryteil 22 weist einen stufenförmig ausgesparten Auflagebereich 60 zur formschlüssigen Aufnahme der Ränder des abdeckenden Abschnitts 8A auf. Der abdeckende Abschnitt 8A rollt von der ersten Umlenkrolle 81 flach in den stufenförmig ausgesparten Auflagebereich 60 ab. Dadurch wird ein Spalt zwischen dem abdeckenden Abschnitt 8A und dem abzudeckenden Bereich 6 minimiert, wodurch insbesondere die Dichtwirkung und auch der optischen Eindruck verbessert ist.

Das zweite Ende des flexiblen Flächengebildes 8 ist an der Verkleidung des zweiten Gantryteils 22 entlang des Verbindungsbereichs 68 befestigt. Der Verbindungsbereich 68 erstreckt sich im Wesentlichen senkrecht zu der Systemachse SA.

Die Fig. 5 zeigt ein Ablaufdiagramm eines Verfahrens zum Abdecken eines abzudeckenden Bereichs 6 einer Gantry 20 eines Computertomographiegeräts 1, wobei die Gantry 20 einen ersten Gantryteil 21, einen zweiten Gantryteil 22, ein flexibles Flächengebilde 8 und eine Spannvorrichtung 84 für das flexible Flächengebilde 8 aufweist, das Verfahren umfassend:
- ein Aufspannen M1 eines abdeckenden Abschnitts 8A des flexiblen Flächengebildes 8 zwischen dem ersten Gantryteil 21 und dem zweiten Gantryteil 22 zum Abdecken eines abzudeckenden Bereichs 6 der Gantry 20 und
- ein Ausführen M2 einer Translationsbewegung des ersten Gantryteils 21 relativ zu dem zweiten Gantryteil 22, wobei der erste Gantryteil 21 einen Rotor 24 mit einem Projektionsdatenakquisitionssystem 27 aufweist und mittels einer Linearführung relativ zu dem zweiten Gantryteil 22 bewegbar gelagert ist.

Mittels der Spannvorrichtung 84 wird eine Längenänderung des abdeckenden Abschnitts 8A bewirkt, wobei die Längenänderung des abdeckenden Abschnitts 8A der Translationsbewegung des ersten Gantryteils 21 relativ zu dem zweiten Gantryteil 22 folgt. Insbesondere ist vorgesehen, dass die die Spannvorrichtung 84 einer Veränderung einer Zugspannung in dem abdeckenden Abschnitt 8A entgegenwirkt.

Mittels der Spannvorrichtung 84 wird ein Übergang 8T zwischen dem abdeckenden Abschnitt 8A und einem zurückgezogenen Abschnitt 8B des flexiblen Flächengebildes 8 ausgebildet, wobei die Längenänderung des abdeckenden Abschnitts 8A erfolgt, indem ein Teilabschnitt des flexiblen Flächengebildes 8 den Übergang 8T zwischen dem abdeckenden Abschnitt 8A und dem zurückgezogenen Abschnitt 8B passiert.

Die Längenänderung des abdeckenden Abschnitts 8A erfolgt durch eine Wickelbewegung des flexiblen Flächengebildes 8 mittels einer Wickelvorrichtung 80 der Spannvorrichtung 84. Der Randbereich 8R des abdeckenden Abschnitts 8A wird mittels der Spannvorrichtung 84 hin zu dem abzudeckenden Bereich 6 der Gantry 20 gedrückt.

## Patentansprüche

1. Computertomographiegerät (1), aufweisend eine Gantry (20) mit einem ersten Gantryteil (21), einem zweiten Gantryteil (22) und einem flexiblen Flächengebilde (8),
- wobei der erste Gantryteil (21) einen Rotor (24) mit einem Projektionsdatenakquisitionssystem (27) aufweist und mittels einer Linearführung derart relativ zu dem zweiten Gantryteil (22) bewegbar gelagert ist, dass eine Translationsbewegung des ersten Gantryteils (21) relativ zu dem zweiten Gantryteil (22) ausgeführt werden kann, wobei die Linearführung ein Schienensystem (52) und ein Wagensystem (54), welches mit dem Schienensystem (52) zusammenwirkt, aufweist, wobei der erste Gantryteil (21) das Wagensystem (54) aufweist und der zweite Gantryteil (22) das Schienensystem (52) aufweist,
- wobei die Gantry (20) eine Spannvorrichtung (84) für das flexible Flächengebilde (8) aufweist und das flexible Flächengebilde (8) einen abdeckenden Abschnitt (8A) aufweist, wobei der abdeckende Abschnitt (8A) zwischen dem ersten Gantryteil (21) und dem zweiten Gantryteil (22) zum Abdecken eines abzudeckenden Bereichs (6) der Gantry (20) aufgespannt ist, wobei der abzudeckende Bereich (6) das Schienensystem (52) umfasst,
- wobei die Spannvorrichtung (84) dazu eingerichtet ist, eine Längenänderung des abdeckenden Abschnitts (8A), welche der Translationsbewegung des ersten Gantryteils (21) relativ zu dem zweiten Gantryteil (22) folgt, zu bewirken, wobei die Spannvorrichtung (84) eine Wickelvorrichtung (80) aufweist, welche für eine Wickelbewegung des flexiblen Flächengebildes (8) eingerichtet ist, wobei die Längenänderung des abdeckenden Abschnitts (8A) mit der Wickelbewegung des flexiblen Flächengebildes (8) einhergeht,
- **dadurch gekennzeichnet, dass** der erste Gantryteil (21) die Spannvorrichtung (84) aufweist.

2. Computertomographiegerät (1) nach Anspruch 1,
- wobei das zweite Ende des flexiblen Flächengebildes (8) an einer Verkleidung des zweiten Gantryteils (22) befestigt ist.

3. Computertomographiegerät (1) nach Anspruch 1 oder 2,
- wobei das flexible Flächengebilde (8) einen zurückgezogenen Abschnitt (8B) aufweist,
- wobei mittels der Spannvorrichtung (84) ein Übergang (8T) zwischen dem abdeckenden Abschnitt (8A) und dem zurückgezogenen Abschnitt (8B) ausgebildet ist,
- wobei die Längenänderung des abdeckenden Abschnitts (8A) erfolgt, indem ein Teilabschnitt des flexiblen Flächengebildes (8) den Übergang (8T) zwischen dem abdeckenden Abschnitt (8A) und dem zurückgezogenen Abschnitt (8B) passiert.

4. Computertomographiegerät (1) nach einem der Ansprüche 1 bis 3,
- wobei das erste Ende des flexiblen Flächengebildes (8) an der Wickelvorrichtung (80) befestigt ist.

5. Computertomographiegerät (1) nach einem der Ansprüche 1 bis 4,
- wobei die Spannvorrichtung (84) dazu eingerichtet ist, einen Randbereich (8R) des abdeckenden Abschnitts (8A) hin zu dem abzudeckenden Bereich (6) der Gantry (20) zu drücken.

6. Computertomographiegerät (1) nach einem der Ansprüche 1 bis 5,
- wobei die Spannvorrichtung (84) eine erste Umlenkrolle (81) für das flexible Flächengebilde (8) aufweist,
- wobei das flexible Flächengebilde (8) zwischen der ersten Umlenkrolle (81) und dem abzudeckenden Bereich (6) der Gantry (20) hindurchgeführt ist,
- wobei die erste Umlenkrolle (81) an einer Außenseite des flexiblen Flächengebildes (8) anliegend das flexible Flächengebilde (8) umlenkt, wenn sich der erste Gantryteil (21) in einem ersten Positionsbereich relativ zu dem zweiten Gantryteil (22) befindet, wobei die Außenseite des flexiblen Flächengebildes (8) von dem abzudeckenden Bereich (6) der Gantry (20) abgewandt ist.

7. Computertomographiegerät (1) nach Anspruch 6,
- wobei die Rollachse der ersten Umlenkrolle (81) und die Linearführung im Wesentlichen senkrecht zueinander ausgerichtet sind.

8. Computertomographiegerät (1) nach Anspruch 6 oder 7,
- wobei die Spannvorrichtung (84) eine Spannvorrichtungs-Tragstruktur (85), ein Schwenklager (86) und einen Schwenkarm (87) aufweist,
- wobei der Schwenkarm (87) mittels des Schwenklagers (86) relativ zu der Spannvorrichtungs-Tragstruktur (85) um eine Schwenkachse (8X) schwenkbar gelagert ist,
- wobei die Rollachse der ersten Umlenkrolle (81) zu der Schwenkachse (8X) im Wesentlichen parallel ist,
- wobei die erste Umlenkrolle (81) von der Schwenkachse (8X) beabstandet an dem Schwenkarm (87) angeordnet ist,
- wobei die Spannvorrichtung (84) ein Federelement (88) aufweist, welches zwischen den Schwenkarm (87) und die Spannvorrichtungs-Tragstruktur (85) gespannt ist und auf den Schwenkarm (87) ein Drehmoment um die Schwenkachse (8X) relativ zu der Spannvorrichtungs-Tragstruktur (85) derart ausübt, dass die erste Umlenkrolle (81) das flexible Flächengebilde (8) hin zu dem abzudeckenden Bereich (6) der Gantry (20) drückt, wenn sich der erste Gantryteil (21) in einem ersten Positionsbereich relativ zu dem zweiten Gantryteil (22) befindet.

9. Computertomographiegerät (1) nach einem der Ansprüche 6 bis 8,
- wobei die Spannvorrichtung (84) eine zweite Umlenkrolle (82) für das flexible Flächengebilde (8) aufweist,
- wobei das flexible Flächengebilde (8) zwischen der ersten Umlenkrolle (81) und der zweiten Umlenkrolle (82) hindurchgeführt ist,
- wobei die zweite Umlenkrolle (82) an einer Innenseite des flexiblen Flächengebildes (8) anliegend das flexible Flächengebilde (8) umlenkt, wenn sich der erste Gantryteil (21) in einem zweiten Positionsbereich relativ zu dem zweiten Gantryteil (22) befindet, wobei die Innenseite des flexiblen Flächengebildes (8) dem abzudeckenden Bereich (6) der Gantry (20) zugewandt ist.

10. Computertomographiegerät (1) nach einem der Ansprüche 1 bis 9,
- wobei ein erstes Ende des flexiblen Flächengebildes (8) an dem ersten Gantryteil (21) derart befestigt ist, dass das erste Ende des flexiblen Flächengebildes (8) der Translationsbewegung des ersten Gantryteils (21) relativ zu dem zweiten Gantryteil (22) folgt,
- wobei ein zweites Ende des flexiblen Flächengebildes (8) an dem zweiten Gantryteil (22) derart befestigt ist, dass während der Translationsbewegung des ersten Gantryteils (21) relativ zu dem zweiten Gantryteil (22) das zweite Ende des flexiblen Flächengebildes (8) relativ zu dem zweiten Gantryteil (22) ruht.

11. Verfahren zum Abdecken des abzudeckenden Bereichs (6) der Gantry (20) des Computertomographiegeräts (1) nach einem der Ansprüche 1 bis 10, wobei die Gantry (20) den ersten Gantryteil (21), den zweiten Gantryteil (22), das flexible Flächengebilde (8) und die Spannvorrichtung (84) für das flexible Flächengebilde (8) aufweist, das Verfahren umfassend:
- ein Aufspannen (M1) eines abdeckenden Abschnitts (8A) des flexiblen Flächengebildes (8) zwischen dem ersten Gantryteil (21) und dem zweiten Gantryteil (22) zum Abdecken eines abzudeckenden Bereichs (6) der Gantry (20) und
- ein Ausführen (M2) einer Translationsbewegung des ersten Gantryteils (21) relativ zu dem zweiten Gantryteil (22), wobei der erste Gantryteil (21) einen Rotor (24) mit einem Projektionsdatenakquisitionssystem (27) aufweist und mittels einer Linearführung relativ zu dem zweiten Gantryteil (22) bewegbar gelagert ist.

12. Verfahren nach Anspruch 11,
- wobei mittels der Spannvorrichtung (84) eine Längenänderung des abdeckenden Abschnitts (8A) bewirkt wird, wobei die Längenänderung des abdeckenden Abschnitts (8A) der Translationsbewegung des ersten Gantryteils (21) relativ zu dem zweiten Gantryteil (22) folgt.

13. Verfahren nach Anspruch 12,
- wobei mittels der Spannvorrichtung (84) ein Übergang (8T) zwischen dem abdeckenden Abschnitt (8A) und einem zurückgezogenen Abschnitt (8B) des flexiblen Flächengebildes (8) ausgebildet wird,
- wobei die Längenänderung des abdeckenden Abschnitts (8A) erfolgt, indem ein Teilabschnitt des flexiblen Flächengebildes (8) den Übergang (8T) zwischen dem abdeckenden Abschnitt (8A) und dem zurückgezogenen Abschnitt (8B) passiert.

14. Verfahren nach Anspruch 12 oder 13,
- wobei die Längenänderung des abdeckenden Abschnitts (8A) durch eine Wickelbewegung des flexiblen Flächengebildes (8) mittels einer Wickelvorrichtung (80) der Spannvorrichtung (84) erfolgt.

15. Verfahren nach einem der Ansprüche 11 bis 14,
- wobei ein Randbereich (8R) des abdeckenden Abschnitts (8A) mittels der Spannvorrichtung (84) hin zu dem abzudeckenden Bereich (6) der Gantry (20) gedrückt wird.

## Claims

1. Computed tomography device (1), having a gantry (20) having a first gantry part (21), a second gantry part (22) and a flexible material sheet (8),
- wherein the first gantry part (21) has a rotor (24) having a projection data acquisition system (27) and is movably mounted by means of a linear guiding arrangement relative to the second gantry part (22) in such a manner that a translational movement of the first gantry part (21) can be performed relative to the second gantry part (22), wherein the linear guiding arrangement has a rail system (52) and a carriage system (54) that works together with the rail system (52), wherein the first gantry part (21) has the carriage system (54) and the second gantry part (22) has the rail system (52),
- wherein the gantry (20) has a tensioning apparatus (84) for the flexible material sheet (8) and the flexible material sheet (8) has a covering section (8A), wherein the covering section (8A) is spanned between the first gantry part (21) and the second gantry part (22) so as to cover a region (6) to be covered of the gantry (20), wherein the region (6) to be covered comprises the rail system (52),
- wherein the tensioning apparatus (84) is configured so as to cause a change in length of the covering section (8A), which follows the translational movement of the first gantry part (21) relative to the second gantry part (22), wherein the tensioning apparatus (84) has a winding apparatus (80) that is configured for a winding movement of the flexible material sheet (8), wherein the change in length of the covering section (8A) accompanies the winding movement of the flexible material sheet (8),
- **characterised in that** the first gantry part (21) has the tensioning apparatus (84).

2. Computed tomography device (1) according to claim 1,
- wherein the second end of the flexible material sheet (8) is fastened to a cladding of the second gantry part (22).

3. Computed tomography device (1) according to claim 1 or 2,
- wherein the flexible material sheet (8) has a retracted section (8B),
- wherein a transition (8T) between the covering section (8A) and the retracted section (8B) is formed by means of the tensioning apparatus (84),
- wherein the change in length of the covering section (8A) occurs in that a part section of the flexible material sheet (8) passes the transition (8T) between the covering section (8A) and the retracted section (8B).

4. Computed tomography device (1) according to one of claims 1 to 3,
- wherein the first end of the flexible material sheet (8) is fastened to the winding apparatus (80).

5. Computed tomography device (1) according to one of claims 1 to 4,
- wherein the tensioning apparatus (84) is configured so as to press an edge region (8R) of the covering section (8A) towards the region (6) to be covered of the gantry (20).

6. Computed tomography device (1) according to one of claims 1 to 5,
- wherein the tensioning apparatus (84) has a first deflecting roller (81) for the flexible material sheet (8),
- wherein the flexible material sheet (8) is guided between the first deflecting roller (81) and the region (6) to be covered of the gantry (20),
- wherein the first deflecting roller (81), abutting an outer side of the flexible material sheet (8), deflects the flexible material sheet (8) if the first gantry part (21) is located in a first position region relative to the second gantry part (22), wherein the outer side of the flexible material sheet (8) is remote from the region (6) to be covered of the gantry (20).

7. Computed tomography device (1) according to claim 6,
- wherein the roller axis of the first deflecting roller (81) and the linear guiding arrangement are oriented essentially perpendicular with respect to one another.

8. Computed tomography device (1) according to claim 6 or 7,
- wherein the tensioning apparatus (84) has a tensioning apparatus supporting structure (85), a pivot bearing (86) and a pivot arm (87),
- wherein the pivot arm (87) is pivotably mounted about a pivot axis (8X) by means of the pivot bearing (86) relative to the tensioning apparatus supporting structure (85),
- wherein the roller axis of the first deflecting roller (81) is essentially parallel to the pivot axis (8X),
- wherein the first deflecting roller (81) is arranged at a distance from the pivot axis (8X) on the pivot arm (87),
- wherein the tensioning apparatus (84) has a spring element (88) that is tensioned between the pivot arm (87) and the tensioning apparatus supporting structure (85) and exerts a torque on the pivot arm (87) about the pivot axis (8X) relative to the tensioning apparatus supporting structure (85) in such a manner that the first deflecting roller (81) presses the flexible material sheet (8) towards the region (6) to be covered of the gantry (20) if the first gantry part (21) is located in a first position region relative to the second gantry part (22).

9. Computed tomography device (1) according to one of claims 6 to 8,
- wherein the tensioning apparatus (84) has a second deflecting roller (82) for the flexible material sheet (8),
- wherein the flexible material sheet (8) is guided between the first deflecting roller (81) and the second deflecting roller (82),
- wherein the second deflecting roller (82), abutting an inner side of the flexible material sheet (8), deflects the flexible material sheet (8) if the first gantry part (21) is located in a second position region relative to the second gantry part (22), wherein the inner side of the flexible material sheet (8) is facing the region (6) to be covered of the gantry (20).

10. Computed tomography device (1) according to one of claims 1 to 9,
- wherein a first end of the flexible material sheet (8) is fastened to the first gantry part (21) in such a manner that the first end of the flexible material sheet (8) follows the translational movement of the first gantry part (21) relative to the second gantry part (22).
- wherein a second end of the flexible material sheet (8) is fastened to the second gantry part (22) in such a manner that during the translational movement of the first gantry part (21) relative to the second gantry part (22) the second end of the flexible material sheet (8) rests relative to the second gantry part (22).

11. Method for covering the region (6) to be covered of the gantry (20) of the computed tomography device (1) according to one of claims 1 to 10, wherein the gantry (20) has the first gantry part (21), the second gantry part (22), the flexible material sheet (8) and the tensioning apparatus (84) for the flexible material sheet (8), the method comprising:
- spanning (M1) a covering section (8A) of the flexible material sheet (8) between the first gantry part (21) and the second gantry part (22) so as to cover a region (6) to be covered of the gantry (20), and
- performing (M2) a translational movement of the first gantry part (21) relative to the second gantry part (22), wherein the first gantry part (21) has a rotor (24) having a projection data acquisition system (27) and is movably mounted by means of a linear guiding arrangement relative to the second gantry part (22).

12. Method according to claim 11,
- wherein a change in length of the covering section (8A) is caused by means of the tensioning apparatus (84), wherein the change in length of the covering section (8A) follows the translational movement of the first gantry part (21) relative to the second gantry part (22).

13. Method according to claim 12,
- wherein a transition (8T) between the covering section (8A) and a retracted section (8B) of the flexible material sheet (8) is formed by means of the tensioning apparatus (84),
- wherein the change in length of the covering section (8A) occurs in that a part section of the flexible material sheet (8) passes the transition (8T) between the covering section (8A) and the retracted section (8B).

14. Method according to claim 12 or 13,
- wherein the change in length of the covering section (8A) occurs due to a winding movement of the flexible material sheet (8) by means of a winding apparatus (80) of the tensioning apparatus (84).

15. Method according to one of claims 11 to 14,
- wherein an edge region (8R) of the covering section (8A) is pressed by means of the tensioning apparatus (84) towards the region (6) to be covered of the gantry (20).

## Revendications

1. Appareil (1) de tomodensitométrie assistée par ordinateur, comportant un portique (20) ayant une première partie (21) de portique, une deuxième partie (22) de portique et une structure (8) plane souple,
- dans lequel la première partie (21) du portique a un rotor (24) ayant un système (27) d'acquisition de données de projection et est, au moyen d'un guidage linéaire, montée mobile par rapport à la deuxième partie (22) du portique, de manière à pouvoir exécuter un mouvement en translation de la première partie (21) du portique par rapport à la deuxième partie (22) du portique, dans lequel le guidage linéaire a un système (52) de rail et un système (54) de chariot, qui coopère avec le système (52) de rail, dans lequel la première partie (21) du portique a le système (54) de chariot et la deuxième partie (22) du portique a le système (52) de rail,
- dans lequel le portique (20) a un dispositif (84) de tension de la structure (8) plane souple et la structure (8) plane souple a un segment (8A) recouvrant, dans lequel le segment (8A) recouvrant est, pour recouvrir une partie (6) à recouvrir du portique (20), tendue entre la première partie (21) du portique et la deuxième partie (22) du portique, dans lequel la partie (6) à recouvrir comprend le système (52) de rail,
- dans lequel le dispositif (84) de tension est agencé pour provoquer une modification de longueur du segment (8A) recouvrant, qui suit le mouvement en translation de la première partie (21) du portique par rapport à la deuxième partie (22) du portique, dans lequel le dispositif (84) de tension a un dispositif (80) d'enroulement, qui est agencé pour un mouvement d'enroulement de la structure (8) plane souple, dans lequel la variation de longueur du segment (8A) recouvrant va avec le mouvement d'enroulement de la structure (8) plane souple,
- **caractérisé en ce que** la première partie (21) du portique a le dispositif (84) de tension.

2. Appareil (1) de tomodensitométrie assistée par ordinateur suivant la revendication 1,
- dans lequel la deuxième extrémité de la structure (8) plane souple est fixée à un habillage de la deuxième partie (22) du portique.

3. Appareil (1) de tomodensitométrie assistée par ordinateur suivant la revendication 1 ou 2,
- dans lequel la structure (8) plane souple a un segment (8B) en retrait,
- dans lequel, au moyen du dispositif (84) de tension, une transition (8T) est formée entre le segment (8A) recouvrant et le segment (8B) en retrait,
- dans lequel la variation de longueur du segment (8A) recouvrant s'effectue en faisant passer un segment partiel de la structure (8) plane souple sur la transition (8T) entre le segment (8A) recouvrant et le segment (8B) en retrait.

4. Appareil (1) de tomodensitométrie assistée par ordinateur suivant l'une des revendications 1 à 3,
- dans lequel la première extrémité de la structure (8) plane souple est fixée au dispositif (80) d'enroulement.

5. Appareil (1) de tomodensitométrie assistée par ordinateur suivant l'une des revendications 1 à 4,
- dans lequel le dispositif (84) de tension est agencé pour pousser une partie (8R) de bord du segment (8A) recouvrant vers la partie (6) à recouvrir du portique (20).

6. Appareil (1) de tomodensitométrie assistée par ordinateur suivant l'une des revendications 1 à 5,
- dans lequel le dispositif (84) de tension a un premier rouleau (81) de renvoi de la structure (8) plane souple,
- dans lequel la structure (8) plane souple passe entre le premier rouleau (81) de renvoi et la partie (6) à recouvrir du portique (20),
- dans lequel le premier rouleau (81) de renvoi s'appliquant à une face extérieure de la structure (8) plane souple renvoie la structure (8) plane souple, lorsque la première partie (21) du portique se trouve dans une première plage de position par rapport à la deuxième partie (22) du portique, dans lequel la face extérieure de la structure (8) plane souple n'est pas tournée vers la partie (6) à recouvrir du portique (20).

7. Appareil (1) de tomodensitométrie assistée par ordinateur suivant la revendication 6,
- dans lequel l'axe du premier rouleau (81) de renvoi et le guidage linéaire sont dirigés sensiblement perpendiculairement l'un à l'autre.

8. Appareil (1) de tomodensitométrie assistée par ordinateur suivant la revendication 6 ou 7,
- dans lequel le dispositif (84) de tension a une structure (85) porteuse de dispositif de tension, un palier (86) de pivotement et un bras (87) de pivotement,
- dans lequel le bras (87) de pivotement est, au moyen du palier (86) de pivotement, monté pivotant autour d'un axe (8X) de pivotement par rapport à la structure (85) porteuse du dispositif de tension,
- dans lequel l'axe du premier rouleau (81) de renvoi est sensiblement parallèle à l'axe (8X) de pivotement,
- dans lequel le premier rouleau (81) de renvoi est monté sur le bras (87) de pivotement à distance de l'axe (8X) de pivotement,
- dans lequel le dispositif (84) de tension a un élément (88) de ressort, qui est tendu entre le bras (87) de pivotement et la structure (85) porteuse du dispositif de tension et applique, au bras (87) de pivotement, un couple autour de l'axe (8X) de pivotement par rapport à la structure (85) porteuse du dispositif de tension, de manière à ce que le premier rouleau (81) de renvoi pousse la structure (8) plane souple vers la partie (6) à recouvrir du portique (20), lorsque la première partie (21) du portique se trouve dans une première plage de position par rapport à la deuxième partie (22) du portique.

9. Appareil (1) de tomodensitométrie assistée par ordinateur suivant l'une des revendications 6 à 8,
- dans lequel le dispositif (84) de tension a un deuxième rouleau (82) de renvoi de la structure (8) plane souple,
- dans lequel la structure (8) plane souple passe entre le premier rouleau (81) de renvoi et le deuxième rouleau (82) de renvoi,
- dans lequel le deuxième rouleau (82) de renvoi, en s'appliquant sur une face intérieure de la structure (8) plane souple, renvoie la structure (8) plane souple, lorsque la première partie (21) du portique se trouve dans une deuxième plage de position par rapport à la deuxième partie (22) du portique, dans lequel la face intérieure de la structure (8) plane souple est tournée vers la partie (6) à recouvrir du portique (20).

10. Appareil (1) de tomodensitométrie assistée par ordinateur suivant l'une des revendications 1 à 9,
- dans lequel une première extrémité de la structure (8) plane souple est fixée à la première partie (21) du portique, de manière à ce que la première extrémité de la structure (8) plane souple suive le mouvement en translation de la première partie (21) du portique par rapport à la deuxième partie (22) du portique,
- dans lequel une deuxième extrémité de la structure (8) plane souple est fixée à la deuxième partie (22) du portique, de manière à ce que, pendant le mouvement en translation de la première partie (21) du portique par rapport à la deuxième partie (22) du portique, la deuxième extrémité de la structure (8) plane souple soit au repos par rapport à la deuxième partie (22) du portique.

11. Procédé de recouvrement de la partie (6) à recouvrir du portique (20) de l'appareil (1) de tomodensitométrie assistée par ordinateur suivant l'une des revendications 1 à 10, dans lequel le portique (20) a la première partie (21) de portique, la deuxième partie (22) de portique, la structure (8) plane souple et le dispositif (84) de tension de la structure (8) plane souple, le procédé comprenant :
- une tension (M1) d'un segment (8A) recouvrant de la structure (8) plane souple entre la première partie (21) du portique et la deuxième partie (22) du portique pour recouvrir une partie (6) à recouvrir du portique (20) et
- une exécution (M2) d'un mouvement en translation de la première partie (21) du portique par rapport à la deuxième partie (22) du portique, dans lequel la première partie (21) du portique a un rotor (24) ayant un système (27) d'acquisition de données de projection et est, au moyen du guidage linéaire, montée mobile par rapport à la deuxième partie (22) du portique.

12. Procédé suivant la revendication 11,
- dans lequel, au moyen du dispositif (84) de tension, on provoque une modification de longueur du segment (8A) recouvrant, dans lequel le segment (8A) recouvrant suit le mouvement en translation de la première partie (21) du portique par rapport à la deuxième partie (22) du portique.

13. Procédé suivant la revendication 12,
- dans lequel, au moyen du dispositif (84) de tension, on forme une transition (8T) entre le segment (8A) recouvrant et un segment (8B) en retrait de la structure (8) plane souple,
- dans lequel la variation de longueur du segment (8A) recouvrant s'effectue en faisant passer un segment partiel de la structure (8) plane souple sur la transition (8T) entre le segment (8A) recouvrant et le segment (8B) en retrait.

14. Procédé suivant la revendication 12 ou 13,
- dans lequel la variation de longueur du segment (8A) recouvrant s'effectue par un mouvement d'enroulement de la structure (8) plane souple au moyen d'un dispositif (80) d'enroulement du dispositif (84) de tension.

15. Procédé suivant l'une des revendications 11 à 14,
- dans lequel on pousse une partie (8R) de bord du segment (8A) recouvrant au moyen du dispositif (84) de tension vers la partie (6) à recouvrir du portique (20).
